Europäisches Patentamt

European Patent Office

Office européen des brevets

(19)

(11) Veröffentlichungsnummer : **0 121 693 B1**

(12) **EUROPÄISCHE PATENTSCHRIFT**

(45) Veröffentlichungstag der Patentschrift :
09.09.87

(51) Int. Cl.⁴ : **C 07 C 37/60, C 07 C 39/08**

(21) Anmeldenummer : **84101596.9**

(22) Anmeldetag : **16.02.84**

(54) Verfahren zur selektiven Herstellung von Dihydroxybenzolen.

(30) Priorität : **11.03.83 DE 3308763**

(43) Veröffentlichungstag der Anmeldung :
**17.10.84 Patentblatt 84/42**

(45) Bekanntmachung des Hinweises auf die Patenterteilung : **09.09.87 Patentblatt 87/37**

(84) Benannte Vertragsstaaten :
**AT BE CH DE FR GB IT LI LU NL SE**

(56) Entgegenhaltungen :
**DE-A- 2 348 957**
**DE-A- 2 410 758**

(73) Patentinhaber : **Degussa Aktiengesellschaft**
**Weissfrauenstrasse 9**
**D-6000 Frankfurt am Main 1 (DE)**

(72) Erfinder : **Drauz, Karlheinz, Dr. Dipl.-Chem.**
**Flurstrasse 5**
**D-6463 Freigericht 1 (DE)**
Erfinder : **Kleemann, Axel, Dr. Dipl.-Chem.**
**Greifenhagenstrasse 25**
**D-6450 Hanau 9 (DE)**

EP 0 121 693 B1

**0 121 693**

**Beschreibung**

Die Erfindung betrifft die Hydroxylierung von Phenol, substituierten Phenolen und deren Derivaten mit Wasserstoffperoxid, und zwar unter Beeinflussung der Produktselektivität bei der Herstellung der möglichen Ortho- sowie der Paraprodukte.

Wichtige Dihydroxybenzole sind Abkömmlinge von Phenol, den Naphtholen, aber auch von Anthracen oder Phenanthren. Sie lassen sich z. B. bei der Herstellung von Farbstoffen, in der Kunststoffproduktion, als Photochemikalie, bei der Herstellung von Pflanzenschutzmitteln verwenden. So wird z. B. Hydrochinon als Para-Hydroxylierungsprodukt von Phenol als Photochemikalie ; Brenzcatechin als das entsprechende Orthoprodukt für Pflanzenschutzmittel eingesetzt. Verschiedene Anwendungsgebiete, wie z. B. als Antioxydantien, sind den Dihydroxyphenolen gemeinsam.

Ihre Herstellung war daher schon lange Gegenstand eingehender Untersuchungen. Die Hydroxylierung wurde sowohl mit Wasserstoffperoxid selbst, wie auch mit Hydroperoxiden, Peroxiden oder auch Persäuren, wie z. B. Perameisen- oder Peressigsäure, durchgeführt. Doch war Wasserstoffperoxid, da es am leichtesten zugänglich war, bevorzugt, da mit Percarbonsäuren, Hydroperoxiden, Peroxiden Nebenreaktionen auftraten. (EP-A-0 027 593).

Stets war bei diesen Hydroxylierungen ein Katalysator anwesend. Dieser Katalysator konnte ein Metalloid, wie Schwefel, Selen, Tellur, Phosphor, Arsen oder Antimon in elementarer Form sein (DE-OS-2 348 957), oder man verwendete Borverbindungen (DE-PS-1 543 830).

Verschiedene Verfahren arbeiteten mit Übergangselementen in Form ihrer Ionen (DE-OS-2 162 552), besonders mit Eisenionen (DE-OS-2 162 589 oder DE-PS-2 407 398) oder Kobaltionen (DE-AS-2 341 743) oder auch mit den entsprechenden Oxiden (US-PS-2 395 638).

Ausserdem wurden starke Säuren, wie Schwefelsäure, Sulfonsäuren (DE-OS-2 138 735, DE-AS-2 410 742, DE-AS-2 410 758, DE-AS-2 462 967) oder Gemische aus Schwefel- und Phosphorsäure (DE-OS-2 138 735) eingesetzt, bzw. wurden in dieser letztgenannten Offenlegungsschrift organische Säuren, wie u. a. Trichloressigsäure oder Weinsäure, genannt.

Die schon genannten Percarbonsäuren dienten ebenfalls als Katalysatoren (FR-PS-1 479 354). Bei den Katalysatoren handelte es sich in allen genannten Fällen um feste oder flüssige Substanzen. Wasserstoffperoxid — als bevorzugtes Oxydationsmittel — wurde meist in wässriger Lösung verschiedener Konzentrationen bis hin zu sehr hohen, explosionsgefährlichen Konzentrationen eingesetzt ; so arbeitete das Verfahren nach der DE-PS-2 064 497 mit Lösungen, die nur noch 5 Gew.-% Wasser enthielten, aber selbst bei diesem höchstkonzentriertem Wasserstoffperoxid lag die Ausbeute an Dihydroxyderivaten nur bei 70 % und sank entsprechend der Verdünnung des Wasserstoffperoxids erheblich ab.

Hinzu kam, dass in diesem wie auch in anderen Verfahren mit einem sehr grossen Überschuss an dem zu hydroxylierenden Phenol gearbeitet werden mußte, um überhaupt die oben angegebene Ausbeute zu erhalten. Wurde dieser Überschuß gesenkt, z. B. von 20 auf 10 Mol pro Mol Wasserstoffperoxid, so sank trotz hoher Konzentration an Wasserstoffperoxid die Ausbeute drastisch.

Bekanntlich erfordern aber derartige Überschüsse an einer Reaktionskomponenten, die ja zurückgeführt werden muß, zusätzliche technische Aufwendungen ; vor allem hinsichtlich der Größe der einzusetzenden Apparaturen.

Da man immer bemüht ist, große Überschüsse an einer Komponente nach Möglichkeit zu vermeiden, wurde versucht, den Einsatz wäßriger Lösungen von Wasserstoffperoxid zu umgehen. So wurden schon verschiedentlich Lösungen von Wasserstoffperoxid in organischen Lösungsmitteln verwendet. Man arbeitete z. B. nach dem Verfahren der DE-PS-2 410 758 bevorzugt mit Wasserstoffperoxidlösungen in Abkömmlingen der Phosphor- oder Phosphonsäure.

Zwar wurden in dieser Patentschrift auch Wasserstoffperoxidlösungen in wasserfreien Lösungsmitteln, wie aliphatische Äther oder Alkylester, genannt, z. B. Diäthyläther, Diisopropyläther, Diisoamyläther, Isoamylacetat ; bevorzugt Isoamylacetat. Diese auch zur Kernhydroxylierung von Phenolen und Phenoläthern geeigneten Wasserstoffperoxidlösungen hatten aber nur einen Gehalt von Wasserstoffperoxid bis zu 6 %. In der Patentschrift wurde auch ausdrücklich darauf hingewiesen, daß bei der Wahl der Konzentrationen an Wasserstoffperoxid keine Explosionsgefahr bestehen solle. Als Katalysator für die Reaktion wurde eine starke Säure, wie Schwefelsäure (100 %ig) oder Fluorsulfonsäure, eingesetzt.

Diese hochkonzentrierten starken Säuren haben aber den Nachteil, daß ihre Abtrennung aus dem Reaktionsgemisch Schwierigkeiten bereitete (DE-AS-2 658 943), vor allem, da ihre Konzentration im Reaktionsgemisch die Reaktionsdauer erheblich beeinflußt.

Die Überschüsse an Phenolen waren zwar gegenüber denen im Verfahren der DE-AS-2 064 497 etwas vermindert, aber dies wog den Nachteil durch die starken Säuren nicht auf.

Eine zusätzliche Schwierigkeit beim Verfahren der DE-PS-2 410 758 bei der Aufarbeitung des Reaktionsgemisches wurde durch die Anwesenheit des nach der Reaktion mit Wasserstoffperoxid gebildeten Wassers hervorgerufen. Da die eingesetzten Lösungsmittel für Wasserstoffperoxid zum Teil höher siedeten als die eingesetzten Phenole und diese oft — vor allem auch Phenol selbst — mit Wasser Azeotrope bildeten, deren Siedepunkte unter denen der organischen Lösungsmittel lagen, war eine einwandfreie Abtrennung der überschüssigen Phenole aus dem Reaktionsgemisch äusserst problema-

2

tisch.

Man ging daher andere Wege und versuchte, zunächst einmal ohne Katalysator, d. h. vor allem ohne die starken Säuren, auszukommen. Da die Katalysatoren in erster Linie für die Aktivierung von Wasserstoffperoxid notwendig waren, wurde im Verfahren der DE-AS-2 658 843 mit organischen Lösungen von Percarbonsäuren gearbeitet. Ein zusätzlicher Katalysator wurde nicht verwendet.

Ganz abgesehen davon, dass das genannte Verfahren eine vollständige Anlage zur Herstellung einer organischen Percarbonsäure, die zunächst aus Wasserstoffperoxid und Carbonsäure gewonnen und darauf durch Extraktion dieser sog. « Gleichgewichtssäure » aus ihrem wäßrigen Medium hergestellt wird, voraussetzt, hatte sich gezeigt, dass eine angeblich gute Selektivität und gute Ausbeute nur durch Anwesenheit zusätzlicher Persäurestabilisatoren möglich war (DE-OS-2 364 181 ; EP-A-0 027 593). Hinzu kam, dass sich diese Selektivität, d. h. das Verhältnis von Ortho- zu Paraprodukten, nur entsprechend dem jeweiligen Hydroxylierungsmittel, z. B. Percarbonsäuren, einstellte und dann nur durch eine Änderung des Hydroxylierungsmittels selbst beeinflusst werden konnte, und auch das nur in sehr bescheidenem Umfang. (DE-AS-2 658 943).

Bei Verwendung des gleichen Hydroxylierungsmittels — aber bei verschiedenen Reaktionstemperaturen — trat praktisch keine Änderung der Selektivität ein, s. Tabelle 1 der DE-PS-2 364 181.

Auch der Zusatz bestimmter, Chelatkomplexe bildender Stoffe schuf hier keine Abhilfe. (DE-PS-2 364 181). Ebenso hatten Änderungen der Reaktionszeit keinen Einfluss auf die Selektivität (EP-A-0 027 593).

Aus dem Obengesagten ergibt sich also, dass sowohl bei der Verwendung von Wasserstoffperoxid selbst oder in Form seiner Perverbindungen, besonders seiner Percarbonsäurentrotz verschiedenartigster Zusätze als Katalysatoren oder Stabilisatoren — kein Verfahren bekannt war, das einerseits befriedigende Ausbeuten, andererseits aber auch eine Einstellung des Verhältnisses der Ortho — zu den Paraverbindungen bzw. von Orthoverbindungen untereinander, wie sie bei der Hydroxylierung substituierter Phenole auftreten, in einem festgelegten System ermöglichte. Die Selektivität stellte in einem vorgegebenen System, dessen wesentliche Parameter das jeweilige Hydroxylierungsmittel und der jeweilige katalysator bzw. die jeweiligen katalysatoren waren, einen feststehenden Faktor dar.

Da die Ortho- und Paraverbindungen bzw. die Orthoverbindungen untereinander als Isomere in ihren Eigenschaften nicht identisch sind und daher ja technisch zum Teil verschiedene Anwendungen finden, war es wünschenswert geworden, die Selektivität bei der Herstellung dieser beiden Isomeren ohne großen technischen Aufwand beeinflussen zu können, d. h., vor allem im Hinblick einer noch stärkeren Gleichgewichtsverschiebung zugunsten einer der beiden Isomeren, besonders z. B. von Brenzcatechin, oder z. B. von 4-Methyl-brenzcatechin. Wesentlich musste dabei sein, dass die vorgegebenen Parameter für ein System nicht verändert werden mussten.

Es wurde nun gefunden, dass sich das Verhältnis von Ortho- zu Paraprodukten bzw. von Orthoverbindungen untereinander, wie sie bei der Hydroxylierung substituierter Phenole auftreten, bei der Herstellung von Dihydroxybenzolen durch Kernhydroxylierung von Phenol oder substituierten Phenolen bzw. deren Phenoläthern unter Verwendung nur eines einzigen Hydroxylierungsmittels, nämlich von Wasserstoffperoxid in wasserfreiem, organischem Lösungsmittel, in Gegenwart ein und desselben Katalysators beeinflussen läßt, wenn man die Umsetzung bei Temperaturen zwischen 40-200 °C mit Lösungen von Wasserstoffperoxid, die wasserfrei sind, d. h. einen Wassergehalt von 0-1 Gew.-%, bevorzugt unter 0,5 Gew.-%, haben, und die mit organischen Lösungsmitteln hergestellt werden, die mit Wasser Azeotrope bilden, deren Azeotropsiedepunkte unter dem Siedepunkt von Wasserstoffperoxid, bezogen auf Normaldruck, liegen, sowie in Gegenwart von Selendioxid durchführt, wobei das Molverhältnis von Phenol bzw. Phenolderivat zu Wasserstoffperoxid zwischen 5 bis 20 : 1, bevorzugt 5 bis 15 : 1, liegt. Als Lösungsmittel für Wasserstoffperoxid, die mit Wasser Azeotrope bilden, kommen z. B. auch in Frage Äther, wie Dioxan, Diisopropyläther, Methyl-tert.-Butyläther.

Besonders geeignet sind Alkyl- oder Cycloalkylester von gesättigten, aliphatischen Carbonsäuren, die eine Gesamtkohlenstoffzahl von 4-8 besitzen.

Bevorzugte Ester dieser letztgenennten Gruppe zum Lösen des Wasserstoffperoxids sind die der Essig- oder Propionsäure, vor allem Essigsäure-n- oder -i-propylester. Auch Gemische der Ester sind verwendbar. Für die Hydroxylierung bevorzugte Lösungen sind solche mit einem Gewichtsverhältnis von Wasserstoffperoxid zu Carbonsäureester von 1 : 4 bis 2 : 1. Dieses Gewichtsverhältnis erreicht man auch bei verdünnteren Lösungen durch Abdestillieren des Carbonsäureesters.

Die genannten Lösungen von Wasserstoffperoxid in Alkyl- oder Cycloalkylestern werden nach dem Verfahren der DE-A-3 225 307.9 gewonnen. Sie können übliche Stabilisatoren enthalten, s. ULLMANN, Enzyklopädie der technischen Chemie, 4. Aufl., Bd. 17, S. 709. Die Konzentrationen des Wasserstoffperoxids in den genannten Lösungen liegen i. a. zwischen 10-70 Gew.-%: Auf jeden Fall müssen die Lösungen homogen sein.

Die für die zur Kernhydroxylierung in Frage kommenden Phenole sind — neben Phenol selbst — substituierte Phenole wie Alkylabkömmlinge, z. B. Kresole, Äthyl- oder Butylphenole, ferner Alkoxyverbindungen, wie Anisol, dann deren Aryl- oder Halogenabkömmlinge, außerdem Arylphenole, wie 4-Hydroxybiphenyl. Auch die Halogen- oder Alkoxyverbindungen des Phenols selbst sind einsetzbar. Von den Phenyläthern seien noch besonders Phenyläthyläther, Phenylisopropyläther oder p-Kresolmethyläther erwähnt.

Die Reaktionstemperatur liegt bevorzugt zwischen 40 und 170 °C.

Der Druck ist für die Umsetzung nicht wesentlich ; im allgemeinen wird Normaldruck angewendet.

Da der erfindungsgemäß einzusetzende Katalysator Selendioxid, der in fester, gepulverter Form, eventuell auch in gelöster Form, eingesetzt wird, sehr wirksam ist, liegt in den meisten Fällen schon nach etwa 10 Minuten ein Umsatz von rund 85 % und mehr, bezogen auf eingesetztes Wasserstoffperoxid, vor. Selendioxid wird in Mengen von 0,000 1 bis 0,5 Mol, bevorzugt von 0,000 5 bis 0,2 Mol, bezogen auf 1 Mol Wasserstoffperoxid, eingesetzt, ganz besonders günstig bei 7 bis 15 : 1.

Durch das erfindungsgemässe Verfahren ist es zum ersten Mal möglich geworden, das Verhältnis der Ortho- zu dem Paraprodukt bzw. das Verhältnis zweier Orthoprodukte zueinander bei Anwendung von ein und demselben Reaktionssystem zu steuern. z. B. liegt das theoretische Verhältnis von Ortho- zu Paraprodukt bei der Hydroxylierung von Phenol bei ca. 2 : 1. Die nach dem Stand der Technik erhaltenen Verhältnisse liegen nun im allgemeinen zwischen Werten von nahezu 1 : 1 bis etwa 3,5 : 1, und es muss hervorgehoben werden, dass die Schwankungsbreite einer der genannten Werte in einem vorgegebenen System sehr gering war und sich nicht zu Gunsten des einen oder anderen Isomeren beliebig verschieben ließ.

Gerade hier setzt die Erfindung ein. Es war wöllig überraschend, dass bei dem erfindungsgemässen Verfahren das Verhältnis der Ortho- zu den Paraprodukten allein durch die Verlängerung der Reaktionszeit beeinflusst werden kann, s. Beispiel 1 und 2.

Im Prinzip ist eine Beeinflussung der entsprechenden Isomeren der substituierten Phenole oder der Phenoläther, z. B. der Kresole, ebenfalls möglich. Nur wird man hier mehr das Augenmerk auf das wirtschaftlich wertvollere Produkt, wie z. B. auf das entsprechende 4-Methylbrenzcatechin, richten. Dieses fällt nach dem erfindungsgemässen Verfahren in ausserordentlicher Reinheit an, s. Beispiel 3. Die Isomerenbildung ist in diesem Fall fast völlig unterdrückt worden, d. h. die Orthoposition zur Hydroxylgruppe des substituierten Phenols ist in bisher nicht bekannter Form bevorzugt worden.

Die Erfindung wird in den folgenden Beispielen näher erläutert. Dabei bedeuten in den Tabellen der Beispiele 1 und 2

BC = Brenzcatechin

HQ = Hydrochinon

S $H_2O_2$ = Ausbeute an Diphenolen, bezogen auf umgesetztes $H_2O_2$

% = Mol%

## Beispiel 1

65,8 g (0,7 Mol) Phenol werden zusammen mit 0,011 g (0,000 1 Mol) Selendioxid auf 110 °C erwärmt. Zu dieser stark gerührten Lösung werden rasch 12,2 g einer 27,9 gew.-%igen Lösung von $H_2O_2$ in Essigsäure-n-propylester zugegeben ($\cong$ 0,1 Mol). Die Temperatur in der Reaktionsmischung steigt auf maximal 147 °C. Nach den in der Tabelle 1 angegebenen Zeiten wird der Umsatz an $H_2O_2$, die Konzentration und das Verhältnis der Dihydroxybenzole und die Selektivität bezüglich umgesetztes $H_2O_2$ bestimmt.

Tabelle 1

| Zeit (min) | Umsatz $H_2O_2$ % | BC g | HQ g | BC/HQ | S $H_2O_2$ |
|---|---|---|---|---|---|
| 5 | 98 | 6,46 | 1,12 | 5,77 | 70,2 |
| 10 | 99,1 | 5,61 | 1,49 | 3,76 | 65,1 |
| 20 | 99,3 | 5,12 | 1,92 | 2,66 | 64,4 |
| 60 | 99,7 | 4,25 | 2,93 | 1,45 | 65,4 |
| 120 | 99,9 | 3,84 | 3,65 | 1,05 | 68,1 |

## Beispiel 2

94,1 g (1,0 Mol) Phenol werden auf 100 °C erwärmt. Zu der gerührten Schmelze setzt man 0,033 g (0,000 3 Mol) Selendioxid und 6,37 g einer 53,4 gew.-%igen Lösung von $H_2O_2$ in Essigsäure-n-propylester zu (0,1 Mol $H_2O_2$). Die Temperatur in der Reaktionslösung steigt auf einen Maximalwert von 148 °C.

Nach den in der Tabelle 2 angegebenen Zeiten wird der Umsatz an $H_2O_2$, die Konzentration und das Verhältnis der Dihydroxybenzole und die Selektivität bezüglich umgesetztes $H_2O_2$ bestimmt.

Tabelle 2

| Zeit (min) | Umsatz $H_2O_2$ % | BC g | HQ g | BC/HQ | S $H_2O_2$ |
|---|---|---|---|---|---|
| 5 | 83,4 | 6,27 | 1,24 | 5,06 | 81,8 |
| 10 | 85,8 | 5,75 | 1,30 | 4,42 | 74,6 |
| 20 | 88,1 | 5,68 | 1,73 | 3,28 | 76,4 |
| 60 | 92,0 | 5,12 | 2,44 | 2,10 | 74,6 |
| 120 | 96,0 | 3,90 | 3,39 | 1,15 | 68,9 |

## Beispiel 3

108,1 g (1,0 Mol) p-Kresol werden auf 100 °C erwärmt, dann werden zu der gerührten Schmelze 0,011 g (0,000 1 Mol) $SeO_2$ und 12,2 g einer 27,9 gew.-%igen Lösung von $H_2O_2$ in Essigsäure-n-propylester zugegeben (0,1 Mol $H_2O_2$). Die Temperatur steigt auf 149 °C. Nach 10 min wird ein Wasserstoffperoxid-Umsatz von 98,8 % bestimmt, im Reaktionsgemisch sind 8,12 g 4-Methylbrenzcatechin entstanden, was einer Ausbeute von 66,2 Mol%, bezogen auf umgesetztes $H_2O_2$, entspricht. 4-Methylresorcin ist in einer Menge von 0,5 Mol%, bezogen auf $H_2O_2$, entstanden (= Spuren, Analysengenauigkeit).

## Beispiel 4

108,1 g (1,0 Mol) o-Kresol werden auf 100 °C erwärmt und gemäss Beispiel 3 umgesetzt. Die Temperatur steigt auf 148 °C. Nach 10 min wird ein $H_2O_2$-Umsatz von 97,9 % bestimmt. Im Reaktionsgemisch sind 7,37 g (59,4 mMol) 3-Methyl-brenzcatechin und 0,66 g (5,3 mMol) 2-Methylhydrochinon entstanden, was einer Gesamtausbeute von 66,1 Mol%, bezogen auf umgesetztes $H_2O_2$, entspricht.

Die Aufbereitung des Reaktionsgemisches ist wesentlich einfacher als es bisher bekannt war.

Da die erfindungsgemäss als Lösungsmittel zu verwendenden Ester niedriger als die zur Umsetzung kommenden Phenole sieden, wird als erstes ein Azeotrop zwischen dem Ester und Wasser abdestilliert. Die Schwierigkeiten einer Wasser-Phenoltrennung, wie sie bisher aufgetreten sind, fallen fort. Dies ist besonders wichtig, da Phenol im Überschuss angewendet wird und wieder zurückgeführt werden muss.

Bei der Aufarbeitung ist es nicht unbedingt notwendig, infolge der extrem niedrigen katalysatorkonzentrationen, vor einer destillativen Trennung eine Abtrennung des Katalysators, beispielsweise durch Neutralisation, vorzunehmen ; das rohe Reaktionsgemisch wird direkt einer Destillation unterworfen.

## Patentansprüche

1. Verfahren zur Herstellung von Dihydroxybenzolen durch Kernhydroxylierung von Phenol oder substituierten Phenolen bzw. deren Phenoläthern mit Wasserstoffperoxid in Form einer wasserfreien organischen Lösung in Gegenwart eines Katalysators, dadurch gekennzeichnet, daß man die Umsetzung bei Temperaturen zwischen 40-200 °C mit Lösungen von Wasserstoffperoxid, die wasserfrei sind, d. h. einen Wassergehalt von 0-1 Gew.-%, bevorzugt unter 0,5 Gew.-%, haben, und die mit organischen Lösungsmitteln hergestellt werden, die mit Wasser Azeotrope bilden, deren Azeotropsiedepunkte unter dem Siedepunkt von Wasserstoffperoxid, bezogen auf Normaldruck, liegen, sowie in Gegenwart von Selendioxid durchführt, wobei das Molverhältnis von Phenol bzw. Phenolderivat zu Wasserstoffperoxid zwischen 5 bis 20 : 1, bevorzugt 5 bis 15 : 1, liegt.

2. Verfahren nach Anspruch 1, dadurch gekennzeichnet, daß man Wasserstoffperoxidlösungen in Alkyl- oder Cycloalkylestern von gesättigten, aliphatischen Carbonsäuren, die eine Gesamtkohlenstoffzahl von 4-8 besitzen, einsetzt. .

3. Verfahren nach Anspruch 1 und 2, dadurch gekennzeichnet, daß man Selendioxid in Mengen von 0,000 1 bis 0,5 Mol bevorzugt von 0,000 5 bis 0,2 Mol, bezogen auf 1 Mol Wasserstoffperoxid, einsetzt.

4. Verfahren nach Anspruch 1 bis 3, dadurch gekennzeichnet, dass man Wasserstoffperoxidlösungen in Essigsäure-n-propylester oder Essigsäure-i-propylester verwendet.

## Claims

1. A process for the production of dihydroxy benzenes by nuclear hydroxylation of phenol or substituted phenols and the phenol ethers thereof with hydrogen peroxide in the form of an anhydrous organic solution in the presence of a catalyst, characterised in that the reaction is carried out at temperatures of between 40 and 200 °C with solutions of hydrogen peroxide which are anhydrous, i.e. have a water content of from 0 to 1 % by weight, preferably less than 0.5 % by weight, and are produced with organic solvents which form with water azeotropes whose azeotropic boiling points lie below the boiling point of hydrogen peroxide, based on normal pressure, and in the presence of selenium dioxide, the molar ratio of phenol or phenol derivative to hydrogen peroxide lying between 5 and 20 : 1, preferably 5 and 15 : 1.

2. A process according to claim 1, characterised in that hydrogen peroxide solutions in alkyl or cycloalkyl esters of saturated aliphatic carboxylic acids having a total carbon number of from 4 to 8 are used.

3. A process according to claims 1 and 2, characterised in that selenium dioxide is used in quantities of from 0.000 1 to 0.5 mol, preferably from 0.000 5 to 0.2 mol, based on 1 mol of hydrogen peroxide.

4. A process according to claims 1 to 3, characterised in that hydrogen peroxide solutions in acetic acid-n-propyl ester or acetic acid-i-propyl ester are used.

**Revendications**

1. Procédé de préparation de dihydrobenzènes par hydroxylation sur le noyau de phénol ou de phénols substitués, ou de leurs éthers phénoliques, avec du peroxyde d'hydrogène sous la forme d'une solution organique anhydre, en présence d'un catalyseur, procédé caractérisé en ce que l'on effectue la réaction à des températures de 40 à 200 °C, avec des solutions de peroxyde d'hydrogène qui sont exemptes d'eau, c'est-à-dire ont une teneur en eau de 0 à 1 % en poids, de préférence inférieure à 0,5 % en poids et qui sont préparées avec des solvants organiques qui forment avec l'eau des azéotropes dont le point d'ébullition azéotropique est inférieur au point d'ébullition du peroxyde d'hydrogène rapporté à la pression normale, ainsi qu'en présence de dioxyde de sélénium, le rapport moléculaire entre le phénol ou ses dérivés et le peroxyde d'hydrogène se situant entre 5 et 20 : 1, ou mieux de 5 à 15 : 1.

2. Procédé suivant la revendication 1, caractérisé en ce que l'on utilise des solutions de peroxyde d'hydrogène dans des esters alcoylés ou cycloalcoylés d'acides carboxyliques aliphatiques saturés qui possèdent un nombre total de carbones de 4 à 8.

3. Procédé suivant les revendications 1 à 2, caractérisé en ce que l'on utilise le dioxyde de sélénium dans des proportions de 0,000 1 à 0,5 mol, ou mieux de 0,000 5 à 0,2 mol, calculé par mole de peroxyde d'hydrogène.

4. Procédé suivant les revendications 1 à 3, caractérisé en ce que l'on utilise des solutions de peroxyde d'hydrogène dans le n-propylester, ou le i-propylester d'acide acétique.